Europäisches Patentamt

European Patent Office

Office européen des brevets

(11) Numéro de publication: **0 409 696 A1**

## (12) DEMANDE DE BREVET EUROPEEN

(21) Numéro de dépôt: 90402009.6

(22) Date de dépôt: 12.07.90

(51) Int. Cl.5: **C12N 5/10, C12N 15/85**

(30) Priorité: 13.07.89 FR 8909559

(43) Date de publication de la demande:
23.01.91 Bulletin 91/04

(84) Etats contractants désignés:
AT BE CH DE DK FR GB IT LI NL SE

(71) Demandeur: UNIVERSITE PARIS VII
2 Place Jussieu
F-75005 Paris(FR)

(72) Inventeur: **Paulin, Denise**
**7 rue de Montreuil**
**F-94300 Vincennes(FR)**

(74) Mandataire: **Rodhain, Claude et al**
**Cabinet Claude Rodhain 30, rue la Boétie**
**F-75008 Paris(FR)**

(54) **Construction immortalisante pour l'immortalisation cellulaire conditionnelle.**

(57) L'invention concerne une construction immortalisante utile notamment pour l'immortalisation cellulaire conditionnelle.

L'objectif de l'invention est de réaliser une construction immortalisante utile pour une immortalisation cellulaire fonctionnant pour tous les types cellulaires et dans toutes les espèces.

Un second objectif est de réaliser une immortalisation conditionnelle pour laquelle le déclenchement ou l'arrêt du processus est contrôlable.

Un troisième objectif est de réaliser une construction immortalisante adaptée à l'injection directe sur les animaux, sans que ceux-ci ne développent de tumeurs, ni ne meurent.

Ces objectifs sont atteints à l'aide d'une construction immortalisante caractérisée en ce qu'elle est constituée d'un fragment d'ADN codant pour un gène immortalisant et d'un fragment d'ADN de la séquence régulatrice de la vimentine, comprenant les sites récepteurs des protéines cellulaires stimulées par un agent d'activation et conditionnant la transcription du gène immortalisant.

FIG. 1

EP 0 409 696 A1

## CONSTRUCTION IMMORTALISANTE POUR L'IMMORTALISATION CELLULAIRE CONDITIONNELLE

La présente invention concerne une construction immortalisante destinée notamment à être utilisée pour l'immortalisation cellulaire conditionnelle. L'immortalisation cellulaire est un état particulier des cellules, dans lequel, celles-ci se multiplient indéfiniment en culture. Lorsque cette immortalisation est conditionnelle, ceci signifie que la multiplication peut être déclenchée ou arrêtée selon que le milieu de culture contient ou non un agent d'activation.

Il est intéressant pour certains laboratoires et centres de recherche de posséder de grandes quantités de cellules en multiplication, par exemple pour isoler des protéines virales, des composés thérapeutiques ou pour élaborer des vaccins.

Dans ce but, on utilise jusqu'à présent trois méthodes d'immortalisation cellulaire.

Un premier procédé d'immortalisation consiste à agir par l'intermédiaire de l'inflammation par un virus transformant, du type adénovirus, virus d'Epstein-Barr ou certains papovavirus tels que le virus SV40 ou le virus du polyome. En effet, on a pu constater que, lorsque des cellules sont infectées par ce type de virus, l'interaction virus-cellule aboutit à la "transformation" cellulaire. A partir du moment ou le génome du virus se trouve dans la cellule, il donne à cette cellule la propriété de croissance indéfinie. On constate, que toutes ces cellules contiennent le gène immortalisant, qui est une protéine se fixant sur l'ADN cellulaire, et qui provoque ainsi sa réplication.

L'inconvénient de ce type de procédé est qu'un type de virus ne peut immortaliser les cellules que d'une ou d'un nombre restreint d'espèces, et non de toutes les espèces, comme cela serait souhaitable. Ainsi par exemple, le virus SV40 transforme, d'une manière efficace, uniquement les cellules de rongeurs et les cellules humaines. De même, les virus de polyome ne transforment efficacement que les cellules de rongeurs et les virus d'Epstein-Barr, que les cellules humaines.

Un second procédé d'immortalisation utilise la transfection avec des constructions contenant un gène transformant, lié à un promoteur viral. Dans ce cas, le virus est débarrassé de sa capside et seul l'ADN pénètre dans la cellule et permet la réplication du gène transformant. En enlevant la capside, on lève la barrière de pénétration, puisqu'il n'est plus nécessaire que le virus trouve un récepteur spécifique sur la membrane cellulaire. Néanmoins, la restriction cellulaire existe encore dans de nombreux cas.

Enfin, on connait un troisième procédé d'immortalisation consistant à utiliser un gène transformant lié à un promoteur cellulaire. On utilise par exemple comme promoteur le gène de l'albumine, mais celui-ci ne permet une expression que dans les cellules hépatiques. D'une manière générale, ce type d'expression est souvent limité à un type cellulaire. Si le promoteur est ubiquitaire, le taux de fonctionnement de l'immortalité est peu élevé et non controlable.

Les techniques de transformation et d'immortalisation ont été notamment décrites dans les articles suivant : "Jat P.S et P.A Sharp. 1986, Large T antigens of simian virus 40 and polyomavirus efficiently establish primary fibroblasts, J. Virol. 59 : 746 - 750" et "Topp W.C., D. Lane and R. Pollack. 1980. Transformation by SV40 and polyoma virus, p 205 -296 InJ. Tooze (ed), Molecular biology of tumor viruses, Cold spring Harbor Labatory, Cold Spring Harbor, N.Y.

On utilise aussi d'autres procédés d'immortalisation, à partir de cellules issues de souris transgéniques dans lesquelles, on introduit dans l'oeuf, des gènes transformants. Cette technique d'introduction de gènes transformants entraîne malheureusement soit une mortalité élevée, soit le développement de tumeurs. De plus, seules certaines cellules ont ensuite pu être immortalisées in vitro.

En conséquence, l'invention a pour objectif de remédier aux inconvénients préalablement cités et de réaliser une construction immortalisante utile pour une immortalisation cellulaire fonctionnant pour tous les types cellulaires et dans toutes les espèces, en particulier dans les cellules humaines.

Un second objectif de cette invention est de réaliser une immortalisation conditionnelle, c'est-à-dire que le déclenchement ou l'arrêt du processus est contrôlable à volonté.

Un troisième objectif de l'invention est de réaliser une construction immortalisante qui puisse être adaptée à l'injection directe sur les animaux, sans que ceux-ci ne développent de tumeurs, ni ne meurent, car le gène est muet chez l'animal et ne devient fonctionnel qu'au cours de la culture cellulaire.

Ces objectifs ainsi que d'autres qui apparaîtront par la suite, sont atteints à l'aide d'une construction immortalisante, caractérisée en ce qu'elle est constituée d'un fragment d'ADN codant pour un gène immortalisant et d'un fragment d'ADN de la séquence régulatrice de la vimentine, comprenant les sites récepteurs des protéines cellulaires stimulées par un agent d'activation et conditionnant la transcription du gène immortalisant.

On comprendra mieux l'invention à la lecture de la description suivante d'un mode préférentiel de réalisation, donné à titre d'exemple illustratif et du dessin joint dans lequel :

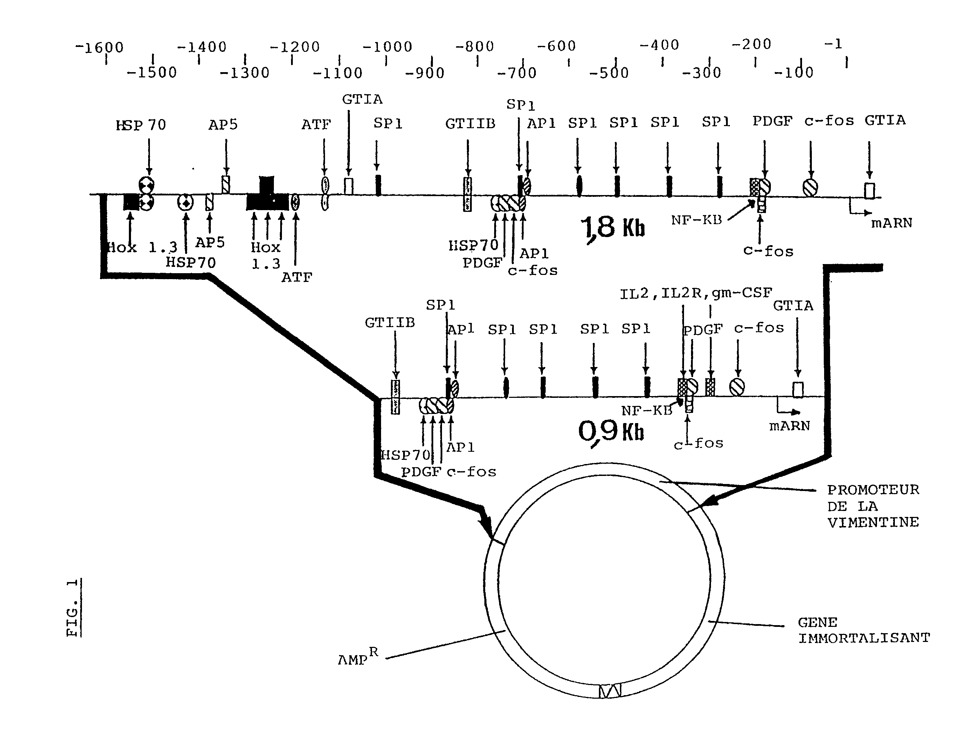

- la figure 1 représente schématiquement le plasmide de la construction immortalisante.

La construction immortalisante selon l'invention est composée de deux parties liées ensemble. Les deux parties sont d'une part le gène immortalisant et d'autre part un fragment d'ADN de la séquence régulatrice de la vimentine.

De façon classique, on peut utiliser, comme gène immortalisant, un gène provenant d'un adénovirus, du virus d'Epstein-Barr, ou du virus du polyome. Dans l'invention, on utilise de préférence l'antigène T du virus SV40.

La vimentine est un polypeptide que l'on retrouve dans les cellules dérivées du mésenchyme. C'est une protéine cytoplasmique qui sert à construire la structure d'une cellule. Ainsi, le gène de la vimentine joue un rôle dans la croissance cellulaire, la différenciation et le développement cellulaire. La vimentime présente l'avantage de posséder de nombreux sites récepteurs de protéines cellulaires pouvant être activées par un agent mitogène. Ces sites sont, de plus, bien placés dans l'espace les uns par rapport aux autres et permettent ainsi aux protéines activées d'y pénétrer facilement et, par conséquent, de déclencher le processus de réplication. Le gène a été isolé et sa séquence nucléotidique a été déterminée.

Dans la construction immortalisante selon l'invention, on utilise de préférence, comme fragment d'ADN de la séquence régulatrice de la vimentine, celui qui correspond au promoteur de la vimentine.

La séquence de bases nucléotidiques du promoteur de la vimentine est donnée ci-après :

```
          CTGCAGTTAA  TCCTTTTCAG  TACACCATAA  ATCTAAATAC
TCTCAAAAAA ACCTGTGCCT (- 1650)
          TTTCAATTGC  TACTAAATCA  CGAGAAGACT  GATTTACATA
GTCTCCTTTT ATCTCCCTTG (- 1590)
          GCGGGTAAGT  ACTCAGCTCT  GCTCGTTACT  AATATTGAAA
CAACAGCCCT TGAATTGAGT (- 1530)
          GATTTCCCTA  GAAAGGTTAA  GGTGACCGAA  TCTGAACACT
CCCTCCATGT CTTGGACACG (- 1470)
          AAGTTTTTTT  TCTGCGTAGA  CAGTTTTATC  CCCTCATCCC
AAGGTCAATT GCACGAATTC (- 1410)
          TTTTGGAAAA  CAGAACCTAT  GGCATTTCCC  AGACAAATCA
CCGTGAACCC TGTACTGTGC (- 1350)
          ATTGCTGTCC  TAAAATTAAC  ACATAAATCT  ATTGCCGCCA
AAGATTCTGT CATTTGTGTT (- 1290)
          ·ACATAATTGC  CTTTCATTTG  AACTCATTAA  TCAAATTGGG
GTTTTTAAGC AACACCTAAT (- 1230)
          TAATTCTTTA  ACTGGCTCAT  ATTAACCTTT  AATGACTTCC
ACCAGGGTAA AAACCACTGA (- 1170)
          TCACTGAGTT  CTATTTTGAA  ACTACGGACG  TCGAGTTTCC
TCTTTCACCC AGAATTTTCA (- 1110)
          GATCTTGTTT  AAAAAGTTGG  GTGTGGTTTC  ATGGGGGGAG
GGGGAAGAGC GAGAGGAGAC (- 1050)
          CAGAGGGACG  GGGGCGGGGA  CTCTGCAAGA  AAAACCTTCC
CGGTGCAATC GTGATCTGGG (- 990)
          AGGCCCACGT  ATGGCGCCTC  TCCAAAGGCT  GCAGAAGTTT
CTTGCTAACA AAAAGTCCGC (- 930)
          ACATTCGAGC  AAAGACAGGC  TTTAGCGAGT  TATTAAAAAC
TTAGGGGCGC TCTTGTCCCC (- 870)
          CACAGGGCCC  GACCGCACAC  AGCAAGGCGA  TGGGCCCAGC
TGTAAGTTGG TAGCACTGAG (- 810)
          AACTAGCAGC  GCGCGCGGAG  CCCGCTGAGA  CTTGAATCAA
TCTGGTCTAA CGGTTTCCCC (- 750)
          TAAACCGCTA  CGAGCCCTCA  ATCGGCGGGA  CAGCAGGGCG
CGGTGAGTCA CCGCCGGTGA (- 690)
          CTAAGCGACC  CCACCCCTCT  CCCTCGGGCT  TTCCTCTGCC
ACCGCCGTCT CGCAACTCCC (- 630)
```

```
        GCCGTCCGAA  GCTGGACTGA  GCCCGTTAGG  TCGATGGACA
GAGGCGCGGG  CCGGAGCAGC  (- 570)
        CCCCCTTTCC  AAGCGGGCGG  CGCGCGAGGC  TGCGGCGAGG
CCTGAGCCCT  GCGTTCCTGC  (- 510)
        GCTGTGCGCG  CCCCCCACCC  CGCGTTCCAA  TCTCAGGCGC
TCTTTGTTTC  TTTCTCCGCG  (- 450)
        ACTTCAGATC  TGAGGGATTC  CTTACTCTTT  CCTCTTCCCG
CTCCTTTGCC  CGCGGGTCTC  (- 390)
        CCCGCCTGAC  CGCAGCCCCG  AGGCCGCCGC  GCACCTCCTC
CCACGCCCCT  TTGGCGTGGT  (- 330)
        GCCACCGGAC  CCCTCTGGTT  CAGTCCCAGG  CGGACCCCCC
CCTCACCGCG  CGACCCCGCC  (- 270)
        TTTTTCAGCA  CCCCAGGGTG  AGCCCAGCTC  AGACTATCAT
CCGGAAAGCC  CCCAAAAGTC  (- 210)
        CCAGCCCAGC  GCTGAAGTAA  CGGGACCATG  CCCAGTCCCA
CGCCCCGGAG  CAGGAAGGCT  (- 150)
        CGAGGCGCCC  CCACCCCACC  CGCCCACCCT  CCCCGCTTCT
CGCTAGGTCC  CGATTGGCTG  (- 90)
        GGGCGCTCCG  CGGCTGGGAT  GGCAGTGGGA  GGGGACCCTC
TTTCCTAACG  GGGTTATAAA  (- 30)
        AACAGCGCCC  TCGGCGGGGT  CCAGTCCTCT  GCCACTCTCG
CTCCGAGGTC  CCCGCGCCAG  (+ 30)
        AGACGCAGCC  GCGCTCCCAC  CACCCACACC  CACCGCGCCC
TCGTTCGCCT  CTTCTCCGGG  (+ 90)
        AGCCAGTCCG  CGCCACCGCC
```

Le promoteur de la vimentine s'étend des bases -1709 à +93. Pour réaliser la construction immortalisante selon l'invention, on peut utiliser notamment, comme fragment d'ADN issu du promoteur de la vimentine, soit le fragment de bases -1709 à +93 (1,8 Kb), soit un fragment de bases -830 à +93 (0,9 Kb). Le choix du fragment est lié au type cellulaire sur lequel on injectera la construction immortalisante, car selon celui-ci, la construction présente une efficacité plus ou moins grande.

La construction immortalisante selon l'invention est de préférence conservée dans un plasmide afin de pouvoir être reproduite facilement. La construction de ce plasmide est décrite ci-après, premièrement lorsque l'on utilise le fragment 0,9 Kb et deuxièmement avec le fragment 1,8 Kb.

Le fragment d'ADN 0,9 Kb du promoteur de la vimentine est clôné dans un plasmide Puc 18 ; il est linéarisé par une enzyme de restriction Xba$_I$ , qui coupe la double hélice d'ADN, ce qui engendre des extrémités 5' phosphates protrudentes. Ensuite, ces extrémités sont complétées par l'action de l'ADN polymérase I, et plus particulièrement le fragment de "kleenow", en présence de 4 dNTP. On obtient ainsi des bouts francs à chaque extrémité. Enfin ce plasmide subit une deuxième coupure par l'enzyme Bam H$_I$ qui engendre une extrémité à bouts francs et une extrémité protrudente.

Par ailleurs, le fragment d'ADN du virus codant pour le gène transformant, par exemple dans le cas du virus SV40, le fragment d'ADN codant pour l'antigène T, est clôné dans un plasmide PBr 327. Ce fragment est linéarisé par l'enzyme de restriction SFi$_l$, engendrant ainsi des extrémités 3$^{'}$OH protrudentes. Ces deux extrémités sont ensuite rendues à bouts francs par l'ADN polymérase I (fragment de kleenow) en présence d'I dNTP. Le plasmide subit alors une deuxième coupure par l'enzyme de restriction Bam H$_l$ , qui permet d'extraire le fragment de 2,7 Kb codant pour l'antigène T du virus SV40. Les extrémités de ce fragment, une protrudente et une à bouts francs, sont compatibles avec le fragment du promoteur de la vimentine préparé précédemment.

Ensuite, on lie le fragment d'ADN du promoteur de la vimentine avec le fragment d'ADN de l'antigène T (tous deux sous forme de plasmide) par l'intermédiaire de la ligase du phage T4. Cette ligation ne peut s'effectuer que d'une seule manière, c'est-à-dire liaison des extrémités à bouts francs ensemble et liaison des extrémités protrudentes ensemble. Ainsi, c'est la préparation de chacun des plasmides (antigène T / PBr 327 d'une part, et 0,9 Kb / Puc 18 d'autre part) avec une extrémité protrudente et une extrémité à bouts francs qui permet d'orienter sélectivement leur sens de ligation. A l'inverse, si ceux-ci étaient préparés avec, par exemple, toutes leurs extrémités à bouts francs, ils pourraient se combiner dans plusieurs sens, ce qui nécessiterait ensuite une étape supplémentaire de séparation des plasmides selon leur sens de ligation. On obtient finalement le plasmide HuProVim 0,9-T.

La préparation du plasmide avec le fragment d'ADN 1,8 Kb du promoteur de la vimentine peut s'effectuer à partir du plasmide HuProVim 0,9-T, de la façon suivante :

Le plasmide HuProVim 0,9-T obtenu précédemment, est coupé sous la double action des enzymes de restriction Sph$_l$ / Xho$_5$. On dispose ainsi d'un plasmide contenant le fragment d'ADN de l'antigène T et un fragment d'ADN de 0,2 Kb du promoteur de la vimentine avec deux extrémités protrudentes.

Par ailleurs, le fragment d'ADN 1,8 Kb du promoteur de la vimentine est clôné dans un plasmide Puc 18. Puis, il est linéarisé et coupé par les enzymes de restriction Sph$_l$ / Xho$_l$ qui permettent d'extraire un fragment d'ADN du promoteur de la vimentine de 1,6 Kb avec deux extrémités protrudentes.

Enfin, ce fragment est cloné dans le plasmide obtenu précédemment (contenant les fragments d'ADN de l'antigène T et du promoteur de la vimentine 0,2 Kb) par l'intermédiaire de la ligase du phage T4. On obtient ainsi le plasmide HuProVim 1,8-T.

Par ailleurs, on prévoit l'addition d'un gène codant pour la résistance à l'ampicilline (fragment Amp$^R$ sur la figure 1) de façon à pouvoir repérer un plasmide selon l'invention dans une boîte de culture en présence d'ampicilline, puisque ce plasmide sera résistant.

La construction immortalisante selon l'invention permet de réaliser une immortalisation conditionnelle lorsqu'elle est employée en combinaison avec un agent d'activation tel qu'un agent mitogène. On peut ainsi initier l'expression du gène immortalisant en introduisant l'agent mitogène dans le milieu de culture.

Par ailleurs, dans certains types de cellules, l'effet mitogène initiateur de la multiplication peut être obtenu par la dissociation des contacts cellulaires.

L'agent d'activation peut consister en l'antigène T lui-même. En effet, on a trouvé d'une manière surprenante que l'antigène T agit sur certains sites du promoteur de la vimentine ; il active en quelque sorte lui-même ce promoteur. Le promoteur active ensuite l'antigène T et ainsi de suite. Par suite, lorsque l'immortalisation est débutée, elle devient constitutive. En outre, sa grande efficacité est due à la constitution même du système.

L'agent mitogène introduit dans le milieu de culture peut être notamment un sérum, un facteur de croissance tel le PDGF, l'interleukine 2, l'interféron, ou la phytohémaglutinine (PHA).

Par exemple, l'introduction d'un agent tel qu'un sérum ou le PDGF dans un milieu de culture contenant un plasmide HuProVim 0,9-T ou HuProVim 1,8-T conditionne l'expression de l'antigène T.

Plus précisément, l'introduction de sérum dans le milieu agit sur les protéines API, c-fos et NF-KB présentes dans la cellule. Celles-ci vont se fixer sur l'ADN du promoteur de la vimentine au niveau notamment des bases -707, -693 et -197, -195, -227 (voir figure 1) permettant au gène codant pour l'antigène T de commencer à reproduire ce gène.

La diminution du taux de sérum dans le milieu de culture ou son absence complète, en permettant la différenciation cellulaire, empêche l'expression de l'antigène T. Ainsi, on a constaté qu'avec des pourcentages de sérum dans le milieu de culture, inférieurs à 1%, l'antigène T n'était plus exprimé.

Notamment dans les cellules épithéliales ou nerveuses capables de se différencier, le promoteur est inactivé après la différenciation et il n'y a donc plus production d'antigène T.

La construction immortalisante selon l'invention peut être utilisée, dans l'objectif d'une immortalisation conditionnelle, selon deux techniques : soit in vitro dans les cellules, soit directement dans l'oeuf de l'animal.

Selon la première variante, la construction immortalisante comprenant le fragment d'ADN de la séquence régulatrice de la vimentine et le gène immortalisant, est injectée dans le noyau des cellules à immortaliser. On injecte ainsi 50 à 200 cellules, puis celles-ci reçoivent le milieu de culture. Après deux semaines, elles sont transférées et cultivées dans les conditions classiques avec 10% de sérum de veau. Le facteur d'activation de la vimentine étant présent, l'antigène T est activé et les cellules sont immortalisées. Au contraire, sauf cas particuliers, dès que l'on arrête d'introduire du sérum dans le milieu de culture, les cellules ne se multiplient plus car le sérum est consommé au cours de la réplication.

Selon la seconde variante, la construction est injectée dans un oeuf fécondé d'animal, tel que souris, lapin, vache, brebis, etc..., puis l'oeuf est réimplanté dans l'utérus de l'animal. On met ensuite en culture les différentes cellules issues de l'embryon. Là encore, lorsque l'on cultive les cellules en présence de sérum, l'antigène T est activé et inversement, en absence de sérum, il est muet. En conséquence, avant la mise en culture, l'antigène T n'est pas activé chez l'animal et celui-ci ne développe pas de tumeur. Cette technique présente l'avantage par rapport à la première, de "stocker" dans un seul animal, plusieurs types cellulaires modifiés, sans avoir à injecter des types cellulaires différents.

On peut également envisager l'utilisation d'un gène immortalisant thermosensible. Ce type de gène présente l'avantage de s'exprimer, in vitro, lorsqu'il est en présence d'un agent d'activation, la température (température ambiante) convenant à la réplication de celui-ci. Mais, in vivo, la température étant souvent supérieure au seuil limite (par exemple 39°C chez le lapin), le gène ne peut plus s'exprimer. L'utilisation d'un gène thermosensible permet ainsi d'écarter tout risque de prolifération incontrôlée, au moment de la réimplantation de l'oeuf dans l'organisme animal -prolifération due notamment à la présence d'agents d'activation dans cet organisme et propres à celui-ci -qui conduirait, par exemple, à la formation de tumeurs.

L'utilisation d'une construction immortalisante selon l'invention conjointement avec un agent d'activation permet d'obtenir de grandes quantités de cellules identiques, qui peuvent être facilement stockées à -180°C.

De plus, ces cellules peuvent être modifiées ou utilisées pour héberger des virus. Ainsi, par exemple, pour obtenir un vaccin, on peut introduire dans les cellules un virus inactivé ou atténué. On obtient ainsi des cellules qui, non seulement sont immortelles, mais qui peuvent de plus stimuler de façon active le système immunitaire et constituer un vaccin.

Une autre application très intéressante de l'immortalisation conditionnelle utilisant la construction immortalisante selon l'invention consiste à effectuer une thérapie par transgénie somatique. Ainsi, on peut prélever des cellules saines d'un organe atteint, les immortaliser in vitro, les multiplier et les réimplanter, de façon à ce que les cellules colonisent l'organe concerné.

De plus, on peut modifier les cellules immortelles en leur injectant un gène "correcteur" d'une anomalie, avant de les réimplanter dans l'organe.

On donnera ci-après un exemple illustratif de l'utilisation de la construction immortalisante selon l'invention.

Exemple :

On prélève sur des rats nouveaux-nés des hépatocytes, qui sont mis en culture sur une couche de collagène. Les cellules sont transfectées avec la solution contenant la construction immortalisante (plasmide de l'antigène T lié au promoteur de la vimentine) à raison de 10 micro g par boîte de culture d'un diamètre de 5 cm. On ajoute aussi dans la boîte de culture du phosphate de calcium et un autre plasmide portant le gène de la résistance à la neomycine. Après 10 heures de contact, le milieu de culture est changé. Les cellules sont ensuite repiquées au bout de 48 heures. Après trois semaines de culture en présence de néomycine, les cellules résistantes sont transférées et clonées. On obtient ainsi des hépatocytes immortalisés.

Bien entendu l'invention n'est pas limitée à l'exemple de réalisation décrit, pour lequel on pourra prévoir des variantes, sans pour autant sortir du cadre de l'invention.

**Revendications**

1) Construction immortalisante, caractérisée en ce qu'elle est constituée d'un fragment d'ADN codant pour un gène immortalisant et d'un fragment d'ADN de la séquence régulatrice de la vimentine, comprenant les sites récepteurs des protéines cellulaires stimulées par un agent d'activation et conditionnant la transcription du gène immortalisant.

2) Construction immortalisante selon la revendication 1, caractérisée en ce qu'elle est constituée d'un fragment d'ADN codant pour un gène immortalisant et d'un fragment d'ADN du promoteur de la vimentine.

3) Construction immortalisante selon la revendication 1 ou 2, caractérisée en ce que le fragment d'ADN de la séquence régulatrice de la vimentine comprend les bases nucléotidiques de -1709 à +93.

4) Construction immortalisante selon la revendication 3, caractérisée en ce que le fragment d'ADN de la séquence régulatrice de la vimentine comprend les bases nucléotidiques de -830 à +93.

5) Construction immortalisante selon l'une quelconque des revendications précédentes, caractérisée en ce que le gène immortalisant est l'antigène T du virus SV40.

6) Construction immortalisante selon l'une quelconque des revendications précédentes, caractérisée en ce que l'agent d'activation est un agent mitogène.

7) Construction immortalisante selon la revendication 6, caractérisée en ce que l'agent mitogène est l'antigène T lui-même.

8) Construction immortalisante selon la revendication 6, caractérisée en ce que l'agent mitogène est un sérum.

9) Construction immortalisante selon la revendication 6, caractérisée en ce que l'agent mitogène est l'interleukine 2.

10) Utilisation de la construction immortalisante selon l'une quelconque des revendications précédentes, pour une immortalisation cellulaire conditionnelle, caractérisée en ce que ladite construction est utilisée conjointement avec un agent d'activation conditionnant la transcription du gène immortalisant.

FIG. 1

**Office européen**
**des brevets**

**RAPPORT DE RECHERCHE**
**EUROPEENNE**

Numéro de la demande

**EP 90 40 2009**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y | BIOLOGICAL ABSTRACTS, vol. 83, 1987, résumé no. 45269, Philadelphia, PA, US; O. HURKO et al.: "Transfection of human skeletal muscle cells with SV40 larte T antigen gene coupled to a metallothionein promotor", & ANN. NEUROL. 20(5): 573-582. 1986 * Résumé * | 1-9 | C 12 N 5/10 C 12 N 15/85 |
| | – – – | | |
| Y | NUCLEIC ACIDS RESEARCH, vol. 17, no. 4, 1989, page 1619, IRL Press; S.R. RITTLING et al.: "AP-1/jun binding sites mediate serum inducibility of the human vimentin promoter" * En entier, en particulier page 1626, ligne 13 - page 1628, ligne 17 * | 1-9 | |
| | – – – | | |
| A | CHEMICAL ABSTRACTS, vol. 107, no. 25, 21 décembre 1987, page 222, résumé no. 230461h, Columbus, Ohio, US; S.R. RITTLING et al.: "Functional analysis and growth factor regulation of the human vimentin promoter", & MOL. CELL. BIOL. 1987, 7(11), 3908-15 * Résumé * | 4,6-8 | |
| | – – – | | **DOMAINES TECHNIQUES RECHERCHES (Int. Cl.5)** |
| P,X | WO-A-8 909 816 (MASSACHUSETTS INSTITUTE OF TECHNOLOGY) * Revendications * | 1-9 | C 12 N |
| | – – – – – | | |

Le présent rapport de recherche a été établi pour toutes les revendications

| Lieu de la recherche | Date d'achèvement de la recherche | Examinateur |
|---|---|---|
| La Haye | 21 novembre 90 | SKELLY J.M. |